# EUROPEAN PATENT SPECIFICATION

(11) **EP 2 256 209 B1**
(45) Date of publication and mention of the grant of the patent: **06.04.2016**
(21) Application number: 09715647.5
(22) Date of filing: 27.02.2009
(51) Int. Cl.: C12P 7/22, C12N 5/04

(54) **COMBINED USE OF METHYL JASMONATE AND CYCLODEXTRINS FOR THE PRODUCTION OF RESVERATROL**
KOMBINIERTE VERWENDUNG VON METHYLJASMONAT UND CYCLODEXTRINEN ZUR HERSTELLUNG VON RESVERATROL
UTILISATION COMBINÉE DE MÉTHYL JASMONATE ET DE CYCLODEXTRINES POUR PRODUIRE DU RÉSVÉRATROL

(30) Priority: 29.02.2008 ES 200800591
(43) Date of publication of application: 01.12.2010
(73) Proprietor: UNIVERSIDAD DE ALICANTE, 03690 Alicante (ES); Universidad De Murcia, 30003 Murcia (ES)
(72) Inventor: BRU MARTINEZ, Roque, E-03690 Alicante (ES); PEDREÑO GARCÍA, Maria Ángeles, E-30003 Murcia (ES); BELCHI NAVARRO, Sarai, E-30003 Murcia (ES); ALMAGRO ROMERO, Lorena, E-30003 Murcia (ES)
(74) Representative: Pons Ariño, Angel
(86) International application number: PCT/ES2009/000108
(87) International publication number: WO 2009/106662

(56) References cited:
- EP-A1- 1 471 141
- WO-A1-2005/012507
- DONNEZ D ET AL: "Bioproduction of resveratrol and stilbene derivatives by plant cells and microorganisms", TRENDS IN BIOTECHNOLOGY, ELSEVIER PUBLICATIONS, CAMBRIDGE, GB, vol. 27, no. 12, 1 December 2009 (2009-12-01), pages 706-713, XP026755683, ISSN: 0167-7799, DOI: DOI:10.1016/J.TIBTECH.2009.09.005 [retrieved on 2009-10-28]
- LIJAVETZKY, D. ET AL.: 'Synergistic Effect of Methyljasmonate and Cyclodextrin on Stilbene Biosynthesis Pathway Gene Expression and Resveratrol Production in Monastrell Grapevine Cell Cultures' BIOMED CENTRAL RES. NOTES, [Online] vol. 1, no. 132, 22 December 2008, Retrieved from the Internet: <URL:http://www. biomedcentral.com/content/pdf/1756-0500-1-1 32. pdf> [retrieved on 2009-06-10]
- TASSONI, A. ET AL.: 'Jasmonates and Na- Orthovanadate Promote Resveratrol Production in Vitis vinifera cv. Barbera Cell Cultures' NEW PHYTOLOGIST vol. 166, 2005, pages 895 - 905
- VEZZULLI, S. ET AL.: 'Methyl Jasmonate Treatment as a Trigger of Resveratrol Synthesis in Cultivated Grapevine' AMERICAN JOURNAL OF ENOLOGY AND VITICULTURE vol. 58, no. 4, 2007, pages 530 - 533
- RIGHETTI, S. ET AL.: 'Resveratrol Production in Vitis vinifera Cell Suspensions Treated with Several Elicitors' CARYOLOGIA vol. 60, no. 1-2, January 2007, pages 169 - 171
- PERVAIZ SHAZIB: "Resveratrol: From grapevines to mammalian biology.", FASEB JOURNAL, vol. 17, no. 14, November 2003 (2003-11), pages 1975-1985, ISSN: 0892-6638
- ADRIAN M ET AL: "Stilbene content of mature Vitis vinifera berries in response to UV-C elicitation", JOURNAL OF AGRICULTURAL AND FOOD CHEMISTRY, vol. 48, no. 12, December 2000 (2000-12), pages 6103-6105, ISSN: 0021-8561

## Description

The present invention relates to the combined use of methyl jasmonate and cyclodextrins to promote resveratrol production by cells capable of synthesizing this compound and the extracellular accumulation thereof.

### PRIOR ART

Stilbenoids are biologically active phenolic compounds with a broad spectrum of antibiotic and pharmacological activity. This type of compound is synthesized by certain plants, including grapevines, as an adaptive mechanism in response to stress, such as ultraviolet irradiation, microbial infection, exposure to heavy metals or ozone treatments. This group of compounds includes t-resveratrol (trans- 3,5,4'-trihydroxystilbene), one of the main constituents of stilbenoids produced in response to stress. The cis-resveratrol isomer is commonly found in extracts of t-resveratrol-producing plants and derivatives. Its presence is due to the slow photoisomerization of the transisomer by irradiation with ultraviolet light.

Based on epidemiological and laboratory studies on humans, animals, animal cells in culture and enzymatic assays, it has been shown that stilbenoids, particularly resveratrol, have favourable effects on health, which makes them desirable for inclusion in human and animal diet.

Resveratrol is present in wine and may be involved in the healthy effects of moderate wine consumption. An increase in the consumption of resveratrol has been proposed as a way of reducing the incidence of cancer and cardiovascular disease in humans. Resveratrol and plant extracts containing resveratrol are also effective in the prevention and treatment of atherosclerosis as anti-inflammatory agents and anti-hyperoxidative agents. Resveratrol produces significant inhibition of the formation of colonic aberrant crypts in a rat model treated with a carcinogenic agent (azoxymethane), suggesting its utility as an inhibitor of tumorigenesis in humans. Resveratrol acts as a promoter of the formation of nitroxides which are vasodilating and anti-platelet aggregation agents.

Taking into account the beneficial role of resveratrol on human and animal health, it is important to have a suitable biological source to enable the collection of resveratrol in sufficient quantities to meet the demand. With this purpose several studies were conducted.

International application WO 0044921 uses the resveratrol synthase gene or a portion of said gene derived from a plant that produces resveratrol and transfers same to a plant that does not produce resveratrol naturally so it may constitutively express same and accumulate resveratrol glucoside derivative in its tissues.

International application WO 9718715 discloses how entire grapevine plants are treated with aluminium chloride, which acts as a resveratrol synthesis elicitor to increase the resveratrol content in the plant and products derived therefrom, such as grapes, must and wine. In another study, suspensions of plant cells from resveratrol-producing plants are stimulated with pieces of fungal cell walls to induce resveratrol synthesis and its accumulation in the culture medium and cells (Liswidowati, et al. 1991. "Induction of stilbene synthase by Botrytis cinerea in cultured grapevine cells" Planta 183:307-314).

Among the compounds proposed as inducers of resveratrol synthesis in potentially resveratrol-producing plants, the cyclodextrins family should be noted. Cyclodextrins are cyclic oligosaccharides of 6-8 alpha-D-glucopyranose residues linked by Alfa-(1-4) bonds. There are many derivatives of this compound obtained by modifying the hydroxyl groups in positions 2,3 and/or 6 of the glucosyl residues of the molecule. Some of the tests performed in this regard are the following:
1) The treatment of grapevine cell suspensions with double-methylated cyclodextrin in positions 2 and 6 of each of the glucosyl residues of the molecule (DIMEB) produces the induction of t-resveratrol synthesis, which is excreted to the culture medium (Morales et al. 1998, "Effect of dimethyl-β-cyclodextrins on resveratrol metabolism in Gamay grapevine cell cultures before and after inoculation with Xylophilus ampelinus" Plant Cell Tiss. Org. Cult. 53:179-187).
2) Randomly methylated cyclodextrins in positions 2, 3 and/or 6 of each of the glucosyl residues of the molecule, with an average methylation degree of 13, or hydroxypropylated cyclodextrins, are capable of inducing t-resveratrol synthesis in cell suspensions of two varieties of grapevine, which is excreted in the culture medium for at least 96 hours, accumulating up to 6000 microg/g fresh weight (ES2190771).
3) When different cell lines of the Vitis genus are treated with cyclodextrins they show an extracellular accumulation of stibenes in the culture medium, particularly t-resveratrol. Depending on the incubation conditions, the ratio of trans and cis resveratrol can be modified. (Roque Bru et al. 2006 "Modified Cyclodextrins are chemically defined glucan inducers of defense responses in grapevine cell cultures" Journay of Agricultural and food chemistry, 54, 65-71.)

These studies show that a doubly-methylated cyclodextrin in positions 2 and 6 of each of the glucosyl residues of the molecule or randomly methylated or hydroxypropylated cyclodextrin, induce extracellular accumulation of t-resveratrol in grapevine cell cultures.

Other studies have demonstrated the possibility of stimulating the production of resveratrol by using methyl jasmonate (MeJA). Methyl jasmonate is an active derivative of jasmonic acid, which along with the latter, is among the compounds that act as defense response regulators in plants under stress. In this sense, some relevant studies are cited below:
1) The treatment of three cell lines of grapevine suspensions with methyl jasmonate leads to the intracellular accumulation of resveratrol mainly (between 90-95%) in the glycosylated form called piceid, with a negligible amount of any form of resveratrol in the extracellular medium. Maximum levels of accumulated piceids are 228 mg/litre. According to cell density data from the study, this would amount to about 11 mg/g dry weight or 500 microg/g fresh weight. Controls show that the piceid accumulated after 14 days is 0.05 mg/g fresh weight. (Krisa et al. (1999) Stilbene production by Vitis vinifera cell suspension cultures: methyl jasmonate induction and 13C biolabelling. J. Nat. Prod. 62: 1688-1690.)
2) In another study, grapevine cell suspensions are treated with methyl jasmonate and the free cis- and trans-resveratrol forms in cell extracts and extracellular medium are analyzed. An increase as regards to the controls is only observed after 6 to 8 days. The differences are around 0.34 microg/g fresh weight. (Tasoni et al (2005) Jasmonates and Naorthovanadate promote resveratrol production in V. vinifera cv. Barbera cell cultures. New Phytologist 166: 895-905). This amount represents approximately 0.006% of that which accumulates in the presence of cyclodextrins (ES2190771). Therefore, the amount of resveratrol in the extracellular medium due to treatment with methyl jasmonate is significantly lower compared to that accumulated due to treatment with cyclodextrins.

Studies to date show that methyl jasmonate induces the intracellular accumulation of the glycosylated form of resveratrol or piceid in grapevine cell culture, yet not significantly affecting the intracellular or extracellular accumulation of the free forms.

### EXPLANATION OF THE INVENTION

The present invention combines cyclodextrin treatment (randomly methylated cyclodextrins or hydroxypropylated cyclodextrin) with methyl jasmonate, in order to promote resveratrol production in potentially resveratrol-producing cells.

Taking into account the prior art described above, it would be expected that, from the combined treatment of grapevine cell suspensions with cyclodextrins and Methyl Jasmonate, the overall accumulation of resveratrol in the culture would consist of the sum of the quantities and forms that accumulate with individual treatments, i.e. around 500 microg/g fresh weight of intracellular piceid and around 6000 microg/g fresh weight of extracellular resveratrol.

Surprisingly and unexpectedly, the combined use of cyclodextrins and Methyl Jasmonate disclosed in this invention:
a. has a synergistic effect on resveratrol production, the extracellular-accumulated t-resveratrol concentrations obtained being much higher than expected.
b. resveratrol accumulation is only extracellular; this fact is advantageous, the extraction and purification of this compound being far simpler if it accumulates in the culture medium rather than intracellularly (lysis and subsequent elimination of cellular residues is not necessary).
c. T-resveratrol is generated almost exclusively (form of interest rather than cis-resveratrol), increasing the trans/cis ratio.

This effect is exemplified in a variety of cases, such as cell lines of different grapevine varieties *(Gamay, Monastrell, Muscat of Hamburg),* with different mineral culture media (MS, Gamborg) at different cell densities (low, high and medium).

Therefore, a first aspect of the present invention is the method of obtaining resveratrol, which comprises the addition of cyclodextrins selected from the group comprising randomly methylated cyclodextrin (RMCD) or hydroxypropylated cyclodextrin (HPCD) and methyl jasmonate to a culture medium, the addition of potentially resveratrol-producing cells selected from the *Vitis vinifera* species to the culture medium, their incubation and the separation of the resveratrol obtained during the incubation from the culture medium.

A preferred embodiment of this invention further comprises the purification of the resveratrol obtained.

In another preferred embodiment of the present invention the Methyl Jasmonate concentration is between 5 and 450 micromoles/litre of culture medium. More preferably the Methyl Jasmonate concentration is between 50 and 150 micromoles/L culture medium and even more preferably between 75 and 125 micromoles/L culture medium.

In particular, the degree of substitution by methyl per unit of glucose (anhydrous) in RMCD is between 1 and 3, more preferably between 1.5 and 2, and even more preferably between 1.6 and 1.9.

On the other hand, the degree of substitution by hydroxypropyl per unit of glucose (anhydrous) in HPCD is between 0.3 to 1.5, preferably between 0.2 and 1.3, more preferably between 0.5 and 1, and even more preferably between 0.6 and 0.9.

In another preferred embodiment of the invention the cyclodextrin concentration is between 6.5 and 130 g/L culture medium. More preferably the cyclodextrin concentration is between 10 and 100 g/L culture medium and even more preferably it is between 50 and 75 g/L culture medium.

In another preferred embodiment of the invention, the cells come from the group of species comprising *Pinus sibirica, P. sylvestris, Gnetum parviflorum, Vitis vinifera, Polygonum cuspidatum, Arachis hypogaea, Eucalyptus, Artocarpus lakoocha, Nothofagus fusca, Phoenix dactylifera, Festuca versuta, Carex fedia, o Veratrum grandiflorum.*

Potentially resveratrol-producing cells mean any cell line capable of producing resveratrol either naturally or after genetic modification.

In another preferred embodiment of the invention the incubation time is between 4 and 288 hours.

Another aspect of the invention relates to a culture medium comprising methyl jasmonate and cyclodextrins.

Another aspect of the present invention is the use of culture media comprising Methyl Jasmonate and cyclodextrins to promote resveratrol production in resveratrol -producing cells.

Lastly, a final aspect of the invention is the combined use of methyl jasmonate and cyclodextrins to promote resveratrol production in resveratrol-producing cells.

Throughout the description and claims the word "comprise" and its variations are not intended to exclude other technical features, additives, constituents or steps. For those skilled in the art, other objects, advantages and characteristics of the invention will emerge partly from the description and partly from the practice of the invention. The following examples and drawings are provided by way of illustration without limiting the scope of the invention.

### BRIEF DESCRIPTION OF THE FIGURES

Fig 1: T-resveratrol production per unit of volume of extracellular medium (g/litre of medium) of *Vitis vinifera Monastrell* over 288 hours of incubation. The average and the standard deviation of four biological replicates are shown.
Fig 2: Chromatographic analysis (HPLC-UV-MS) of *Vitis vinifera Monastrell Verde* media after incubation for 96 hours. A representative experiment of four biological replicates is shown.
   A and B: Incubated with RMCD at a concentration of 62.5 g/l.
   C and D: Incubated with RMCD at a concentration of 62.5 g/l and methyl jasmonate 100 micromolar.
   A and C are chromatographic absorption records at 306 nm.
   B and D are chromatographic records of ion m/z=229 in positive mode.

   The peaks corresponding to trans and cis forms are indicated.

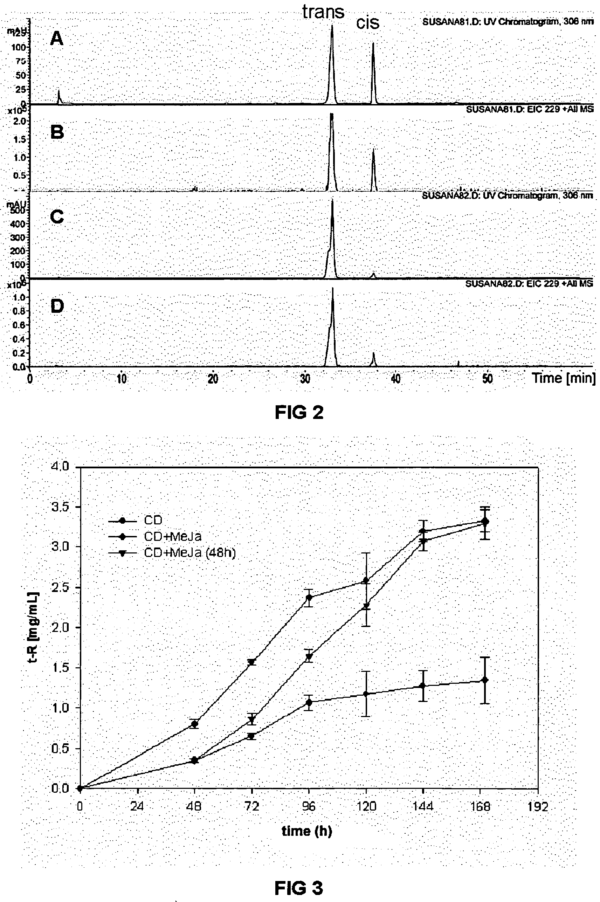

Figure 3: Effect on the kinetics of extracellular resveratrol accumulation of sequentially adding RMCD followed by MeJa to a *Vitis vinifera Gamay variety* culture. Addition of RMCD to the culture and subsequent addition of MeJa after 48 hours (CD + MeJa (48h)), compared to the effect of its combined addition (CD + MeJa) or exclusively using RMCD (CD). The average and the standard deviation of four biological replicates are shown.

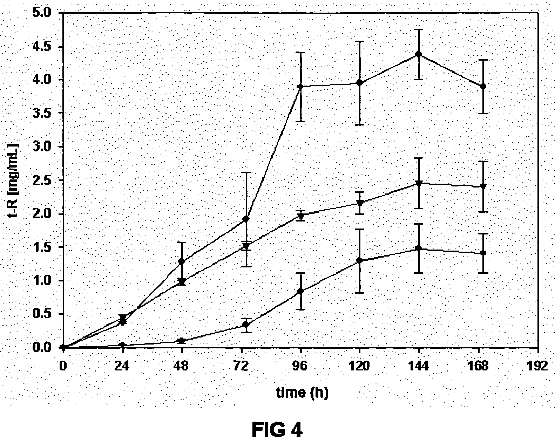

Figure 4: Effect on the kinetics of extracellular resveratrol accumulation of sequentially adding MeJa followed by RMCD to a *Vitis vinifera Gamay variety* culture.

Black circles: culture pre-incubated for 48 hours in culture medium and supplemented at the end of that time with RMCD to the final concentration of 62.5 g/l.

Gray circles: culture pre-incubated for 48 hours in culture medium and supplemented at the end of that time with RMCD to the final concentration of 62.5 g/l and MeJA to the final concentration of 100 micromolar.

Triangles: culture pre-incubated for 48 hours in culture medium with MeJA and supplemented after that time with 100 micromolar RMCD to the final concentration of 62.5 g/l.

Time 0 indicates when the cultures are supplemented with RMCD; there is no resveratrol accumulation previously. The average and standard deviation of four biological replicates are shown.

### DETAILED DESCRIPTION OF EMBODIMENTS

The invention is illustrated hereunder by means of tests conducted by the inventors, which show the synergistic effect of different varieties of resveratrol-producing cells on the joint use of Methyl Jasmonate and different types of cyclodextrins in t-resveratrol production.

### Preparation and maintenance of plant material

### Vitis vinifera var, Gamay cell line

The Vitis vinifera var. Gamay cell line was established by J. C. Pech in 1978 (ENSAT, Toulouse, France) from immature fruits of grapevine from the cultivar Gamay Fréaux.

The calli were maintained at 25°C under a photoperiod of 16 hours of light (6 W/m²) and 8 h of darkness, in 250 ml-capacity flasks containing 80 ml of the medium G20, consisting of Gamborg B5 basal medium (Gamborg et al. 1968, Experimental Cell Research 50:151-158) supplemented with Morel vitamins (Morel, G. 1970. "Le problème de la transformation tumorale chez les végétaux". Physiologie Végétale, 8:189-204), casein hydrolyzate (0.25 g/l), sucrose (20 g/l) as carbon source and alpha-naphthaleneacetic acid (0.1 mg/l) and kinetin (0.2 mg/l) as hormones. This medium is adjusted to pH 6 and is sterilized by applying moist heat (autoclave) for 20 min at a pressure of 1.2 atm, acquiring solid consistency by adding purified agar (8 g/l), when the medium cools.

Cell suspensions were started, from friable calli obtained, in 250 ml-capacity flasks containing 80 ml G20 medium without agar. Cells in liquid medium were kept in an orbital shaker at 100 rpm, at 25°C under a photoperiod of 16 hours of light (6 W/m²) and 8 h of darkness.

### Vitis vinifera var. Monastrell and var. Muscat of Hamburg cell lines

*Vitis vinifera var. Monastrell* and *var. Muscat of Hamburg* cell lines were established from immature fruits of grapevine from cultivars Monastrell and Muscat of Hamburg respectively.

Unripe grapes of about 5 mm in diameter were surface sterilized by immersion in a solution of 7% calcium hypochlorite for 15 minutes. After this time and always under sterile conditions, the grapes were washed 3 times with sterile bidistilled water, seeds were removed and the grapes were divided into 4 portions. These portions (explants) were placed in a Petri dish in darkness at 25°C until microcalli appeared. The Petri dish contained a solid MS2 culture medium, based on that described by Murashige and Skoog (Murashige, T and Skoog, F. 1962. "A revised medium for rapid growth and bioassays with tobacco tissue cultures". Physiologia Plantarum 15:473-497), supplemented with Morel vitamins, casein hydrolyzate (0.25 g/l), sucrose (30 g/l) as carbon source, alpha-naphthaleneacetic acid (0.2 mg/l) and kinetin (0.4 mg/l) as hormones, 8 g/l of purified agar and adjusted to pH 6.

The microcalli obtained were transferred to 250 ml-capacity flasks containing 80 ml of this same solid culture medium for callus development in darkness at 25°C. The calli were maintained by periodic subculture in the same medium and conditions.

Cell suspensions were started, from friable calli in 250 ml-capacity flasks containing 80 ml of the G20 medium, or of the MS2 medium in the absence of agar. The cells in liquid medium were maintained in an orbital shaker at 100 rpm, at 25°C in the dark.

### Cell elicitation

In sterile conditions, each stimulation trial took between 20 and 200 g fresh weight of cells that had been previously washed with fresh medium and filtered. These cells were transferred into a flask of between 250 and 2000 ml capacity and resuspended by adding 100 to 1000 ml of sterile fresh medium supplemented with:
∘ randomly methylated β-cyclodextrin only with a degree of methyl substitution of between 1.6 and 1.9 (RMCD) at a concentration of between 6.5 and 130 g/L or
∘ randomly hydroxypropylated with a degree of hydroxypropyl substitution of between 0.6 and 0.9 (HPCD) at a concentration of between 6.5 and 130 g/L, or
∘ one of the aforementioned cyclodextrins (RMCD) or (HPCD) at a concentration of between 6.5 and 130 g/L and methyl jasmonate at a concentration of between 5 and 450 micromolar.

The methyl jasmonate dissolved in ethanol is sterilized apart by filtration, and then mixed with the rest of the sterile medium. The final ethanol concentration in the culture medium is 0.2% volume.

The flasks were incubated under the same conditions as described above to maintain them in liquid medium for periods ranging from 24 to 256 hours.

### Sampling and sample preparation for analysis

Aliquots were periodically collected for analysis from cultures incubated with elicitors. The cells were separated from the medium by filtration under a slight vacuum, collecting the cells and filtrate separately. The filtrate was used for HPLC analysis.

### Trans-resveratrol analysis in the extracellular medium

An aliquot of the extracellular medium recovered was diluted in water, filtered through a 0.2µm Anopore filter, and 20µl of the filtrate were analyzed by HPLC by injection into a LiChrospher 100 RP-18 column (250x4 mm, particle size, 5µm). Two kinds of solvents were used as a mobile phase: solvent A, 0.05% trifluoroacetic acid in water and solvent B, 0.05% trifluoroacetic acid in methanol: acetonitrile 60:40 vol/vol. The sample is eluted at a flow rate of 1 ml/min of the following mixture of solvents: 0 min. 10% B, 5 min, 15% B; 40 min, 35% B; 45 min, 65% B; 50 min, 65% B; 55 min 10% B; and a column temperature of 35°C (Dalluge et al., 1998, J. Chromatogr. A 793:265-274). The column eluate is passed through a UV-vis detector and then through a mass spectrometer with electrospray ionization source at atmospheric pressure.

The retention time of t-resveratrol and the detector response to the concentration of same (calibration curve for quantification) was determined using commercial t-resveratrol with >99% purity. The presence of resveratrol and other stilbenoids was detected at 306nm and its identity was confirmed by on-line mass spectrometry.

For routine t-resveratrol analysis, an aliquot of filtrate was diluted with fresh medium and its ultraviolet absorption spectrum was recorded using a spectrophotometer using the fresh medium as reference. The t-resveratrol concentration in the supernatant was estimated using a molar extinction coefficient at 306 nm of 26800 M⁻¹cm⁻¹ (Siemann, EH and Creasy, L.L. 1992, "Concentration of the phytoalexin resveratrol in wine" Am. J. Enol. Vitic. 43:49-52).

### EXAMPLE 1: Determination of the optimal concentration of Methyl Jasmonate (MeJa) at variable concentrations of randomly methylated beta-cyclodextrin (RMCD)

The *Vitis vinifera var. Monastrell* cell suspension was treated:
a. with methyl jasmonate at concentrations between 5 and 450 micromoles per litre of culture medium
b. with randomly methylated beta-cyclodextrin (RMCD) at a concentration of 62.5 g/litre and methyl jasmonate (MeJa) at concentrations between 5 and 450 micromoles per litre of culture medium,
c. control without randomly methylated beta-cyclodextrin (RMCD) or methyl jasmonate (MeJA)
d. control without randomly methylated beta-cyclodextrin (RMCD) or methyl jasmonate (MeJA) but with 0.2% v/v ethanol.

The experiments were carried out in triplicate in 250-ml flasks containing 100 ml of culture. After 96 hours, the cells and the culture of each flask were collected separately, the cells were weighed and the total amount of t-resveratrol in the extracellular medium was determined.

The result of the experiment is shown in Table A. The small amounts of extracellular t-resveratrol accumulated in treatments with methyl jasmonate alone are not statistically different from those induced by ethanol alone.

The accumulation increase by combining cyclodextrins and methyl jasmonate is statistically significant compared to treatment with cyclodextrins alone and much greater than the sum of the amounts accumulated with the elicitors used separately.

The optimal concentration under these conditions is 100 micromoles/litre of culture medium.

**TABLE A: Production of t-resveratrol per unit of weight of Vitis vinifera Monastrell cells based on the concentration of MeJA, with and without RMCD, after 96 hours of incubation.**

| **Treatment** | **tR (mg/g fresh weight)** |
|---|---|
| Control | Not detected |
| EtOH | 0.08±0.01 |
| MJ5 | 0.06±0.01 |
| MJ25 | 0.16±0.03 |
| MJ100 | 0.19±0.04 |
| MJ270 | 0.01±0.01 |
| MJ450 | Not detected |
| CD | 7.41 ±1.12 |
| CDMJ5 | 14.63±1.21 |
| CDMJ25 | 15.16±1.57 |
| CDMJ100 | 21.78±1.83 |
| CDMJ270 | 11.22±1.66 |
| CDMJ450 | 9.86±1.02 |

| | |
|---|---|
| Control: culture medium without elicitors; EtOH: culture medium without elicitors with 0.2% v/v ethanol; MJ: Treatment with methyl jasmonate at the concentration indicated by the number in micromolar CD: Treatment with cyclodextrin RMCD at a concentration of 62.5 g/l CDMJ: Treatment with cyclodextrin RMCD at a concentration of 62.5 g/l and methyl jasmonate at the concentration indicated by the number in micromolar | |

### EXAMPLE 2: Determination of the optimal cell density of the culture

The cell density of *Vitis vinifera var. Monastrell* suspensions during stimulation was handled by adding different amounts of fresh filtered cells at a constant volume of culture medium.

Suspensions with densities of between 25 and 75% of packed cell volume (volume occupied by cells in the total culture volume) were treated with:
a. randomly methylated beta-cyclodextrin (RMCD) at a concentration of 62.5 g/l
b. randomly methylated beta-cyclodextrin (RMCD) at a concentration of 62.5 g/l and methyl jasmonate (MeJA) at a concentration of 100 micromoles/l.

The experiments were carried out in triplicate in 250-ml flasks containing 100 ml of culture.

After 96 hours, the cells and the medium of each flask were collected separately, the cells were weighed and the total amount of t-resveratrol in the extracellular medium was determined.

The result of the experiment is shown in Table B. When the culture was stimulated either with RMCD alone or combined with MeJA, a negative correlation was given between the extracellular accumulation of t-resveratrol per unit of biomass and the total biomass, so that the optimal cell density is the lowest (25% PCV).

However, the decline in production per unit of biomass was less pronounced when combining RMCD with MeJA.

**TABLE B: Production of t-resveratrol (tR) per unit of weight of Vitis vinifera Monastrell cells based on cell density measured as packed cell volume (PCV) after 96 hours of incubation.**

| **PCV (%)** | **tR (mg/g fresh weight)** |
|---|---|
| 25CD | 4.96±0.91 |
| 50CD | 1.52±0.33 |
| 75CD | 0.51 ±0.06 |
| 25CDMJ | 9.39±0.32 |
| 50CDMJ | 6.65±0.74 |
| 75CDMJ | 5.55±0.91 |

| | |
|---|---|
| 25, 50 and 75: percentage of packed cell volume CD: RMCD treated with a concentration of 62.5 g/l; CDJ: treated with RMCD (62.5 g / l) and methyl jasmonate 100 micromolar. | |

### EXAMPLE 3: Observation of the synergistic effect of the combined use of RMCD and Methyl Jasmonate

*Vitis vinifera var Gamay* suspensions with a density of 25% PCV were treated:
a. with randomly methylated beta-cyclodextrin (RMCD) at a concentration of 62.5 g/litre,
b. with randomly methylated beta-cyclodextrin (RMCD) at a concentration of 62.5 g/litre and 0.2% ethanol,
c. with randomly methylated beta-cyclodextrin (RMCD) at a concentration of 62.5 g/litre and methyl jasmonate (MeJA) at a concentration of 100 micromoles/l
d. control without randomly methylated beta-cyclodextrin or methyl jasmonate, or ethanol.

The experiments were carried out in triplicate in 250-ml flasks containing 100 ml of culture. After 96 hours, the cells and the medium of each flask were collected separately, the cells were weighed and the total amount of t-resveratrol in the extracellular medium was determined.

The result of the experiment is shown in Table C. When the culture is stimulated with RMCD alone, there is a significant extracellular tR accumulation per unit of biomass; the presence of ethanol together with RMCD has a negative effect, reducing to about half the tR level accumulated. By contrast, the combination of RMCD with MeJA, although also containing 0.2% ethanol, results in an increase of 4.5 times in the extracellular tR accumulation. Therefore, the synergistic effect of RMCD and MeJA observed in the *Monastrell* variety also occurs in *Gamay.*

**TABLE C: T-resveratrol production per unit of Vitis vinifera Gamay cell weight after incubation for 96 hours.**

| **Treatment** | **tR (mg/g fresh weight)** |
|---|---|
| Control | Not detected |
| RMCD | 1.97 ± 0.32 |
| RMCD + 0.2% Ethanol | 0.79 ± 0.09 |
| RMCD + MJ | 10.06 ± 2.19 |

| | |
|---|---|
| RMCD; treatment with randomly methylated beta-cyclodextrin at a concentration of 62.5 g/l; MJ: treatment with methyl jasmonate 100 micromolar. | |

### EXAMPLE 4: Effect of the mineral medium, the type of cyclodextrin and culture cell density (Vitis vinifera var. Muscat of Hamburg)

Cell suspensions of *Muscat of Hamburg* grapevines that had been established in different mineral mediums, particularly Gamborg B5 (G) and Murashige-Skoog (MS2), were elicited in the media indicated under different conditions of cell density and type of cyclodextrin (randomly methylated beta-cyclodextrin or RMCD and hydroxypropylated cyclodextrin or HPCD) in the absence or presence of methyl jasmonate.

The experiments were carried out in triplicate in 250-ml flasks containing 100 ml of culture. After 96 hours, the cells and medium of each flask were collected separately, the cells were weighed and the total amount of t-resveratrol in the extracellular medium was determined.

The result of the experiment is shown in Table D.

Regardless of the type of cyclodextrin tested, the cell density of the culture or the composition of the mineral medium, RMCD and HPCD cyclodextrins both induced extracellular t-resveratrol accumulation at a similar level, methyl jasmonate alone did not induce extracellular t-resveratrol accumulation, whereas combined with RMCD or HPCD the accumulation was between 1.5 and 9.4 times compared to that achieved with cyclodextrin alone, although in most conditions the increase in accumulation is around three times.

The synergistic effect on extracellular t-resveratrol accumulation obtained by combining RMCD or HPCD cyclodextrins with MeJA is also found in the *Muscat of Hamburg* variety, and regardless of the composition of mineral medium and cell density.

**TABLE D: Production of t-resveratrol per unit weight of cells ± standard deviation (mg/g fresh weight) of Vitis vinifera Muscat of Hamburg, depending on the mineral medium used, cell density and type of cyclodextrin, after incubation for 96 hours.**

| MEDIU M | Density | Treatment | | | | | |
|---|---|---|---|---|---|---|---|
| | | Control | MeJA | RMCD | RMCD+MeJ A | HPCD | HPCD+MeJ A |
| G | low | 0.00±0.0 0 | 0.01±0,0 1 | 2.53±0.0 9 | 7.23±1.37 | 4.98±1.2 4 | 7.57±2.96 |
| | high | 0.00±0.0 0 | 0.00±0.0 0 | 1.97±0.1 7 | 5.12±0.68 | 1.20±0.2 1 | 3.67±0.43 |
| MS2 | low | 0.00±0.0 0 | 0.00±0.0 0 | 2.37±0.6 9 | 5.61±1.54 | 1.82±0.4 4 | 5.00±1.27 |
| | high | 0.00±0.0 0 | 0.00±0.0 0 | 1.66±0.2 9 | 4.56±0.74 | 0.73±0.3 4 | 2.82±0.00 |
| GMS2 | low | 0.00±0.0 0 | 0.00±0.0 0 | 6.12±0.3 4 | 10.75±3.16 | 5.11±0.6 4 | 7.85±1.11 |
| | high | 0.00±0.0 0 | 0.00±0.0 0 | 1.61±0.5 6 | 9.75±0.07 | 1.09±0.1 4 | 10.28±1.74 |

| | | | | | | | |
|---|---|---|---|---|---|---|---|
| Low Density: 15 g fresh weight/100 ml cell suspension; High Density: 37 g fresh weight/100 ml cell suspension; MeJA: treatment with 100 micromoles/l Methyl Jasmonate; RMCD: treatment with 62.5g/l of randomly methylated beta-cyclodextrin; HPCD: treatment with 69 g/l hydroxypropylated cyclodextrin; G: Gamborg B5 medium; MS2: Murashige-Skoog medium; GMS2: Gamborg B5 medium with the hormonal composition of MS2. | | | | | | | |

### EXAMPLE 5: Determination of incubation time and t-Resveratrol accumulation kinetics after grapevine cells elicitation with randomly methylated beta-cyclodextrin (RMCD) and methyl jasmonate (MeJA).

Cell suspensions of *Vitis vinifera var. Monastrell were* treated:
a. with methyl jasmonate (MeJA) at a concentration of 100 micromoles/litre
b. with randomly methylated beta-cyclodextrin (RMCD) only at a concentration of 62.5 g/litre
c. with randomly methylated beta-cyclodextrin (RMCD) at a concentration of 62.5 g/litre and methyl jasmonate MEJA at a concentration of 100 micromoles/litre
d. controls without randomly methylated beta-cyclodextrin (RMCD) or methyl jasmonate (MeJA).

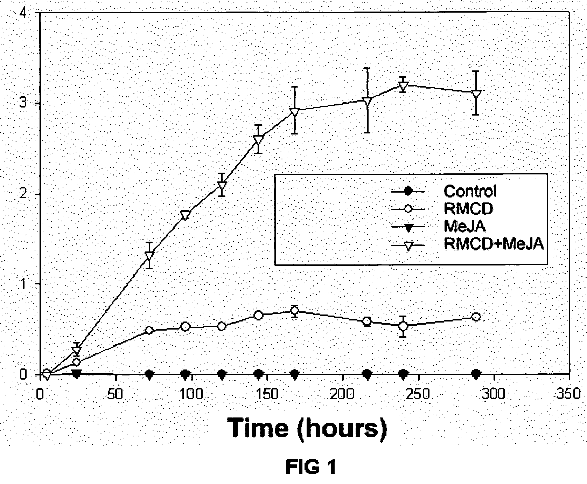

Samples were taken periodically and the cells and medium were collected separately, the cells were weighed and the total amount of t-resveratrol in the extracellular medium was determined. The experiments were carried out in triplicate in 250-ml flasks containing 100 ml of culture. The result of the experiment is shown in Table E and Figure 1.

The presence of t-resveratrol in the controls was never detected and upon treatment with MeJA, it is only detected after 24 hours, to no longer be detected during the remaining incubation time. In the presence of RMCD, t-resveratrol accumulates faster during the first 72 hours, while the accumulation decelerates almost to a stop after 144 hours. In the presence of both RMCD and MeJA, the accumulation is rapid and sustained during the first 168 hours, to decelerate thereafter until almost stopping. Thus, the highest accumulation in the presence of RMCD and MeJA is due to two factors:
- a higher accumulation rate, rises from 0.16 to 0.45 g/litre.day and
- a longer sustained accumulation period, from 72 extends to 168 hours

**Table E: T-resveratrol production per unit of volume of extracellular medium ± standard deviation (g/litre medium) of Vitis vinifera Monastrell over 288 hours of incubation.**

| **time (hours)** | **Control** | **RMCD** | **MeJA** | **RMCD+MeJA** |
|---|---|---|---|---|
| 4 | Not detected | 0.006±0.005 | Not detected | 0.002±0.001 |
| 24 | Not detected | 0.132±0.007 | 0.016±0.014 | 0.280±0.078 |
| 72 | Not detected | 0.486±0.025 | Not detected | 1.319±0.149 |
| 96 | Not detected | 0.525±0.028 | Not detected | 1.769±0.045 |
| 120 | Not detected | 0.531±0.016 | Not detected | 2.099±0.125 |
| 144 | Not detected | 0.650±0.005 | Not detected | 2.603±0.158 |
| 168 | Not detected | 0.699±0.065 | Not detected | 2.915±0.259 |
| 216 | Not detected | 0.582±0.045 | Not detected | 3.032±0.355 |
| 240 | Not detected | 0.527±0.119 | Not detected | 3.203±0.085 |
| 288 | Not detected | 0.629±0.005 | Not detected | 3.106±0.237 |

| | | | | |
|---|---|---|---|---|
| MeJA: treatment with 100 micromoles/l of MeJA; RMCD: treatment with 62.5g/l of RMCD. | | | | |

### EXAMPLE 6: Elicitation of grapevine cells (Vitis vinifera var. Monastrell verde) with RMCD and MeJA.

High density (48 g fresh weight/100 mL) *Vitis vinifera Monastrell verde* suspensions were treated with:
(i) RMCD (62.5 g/litre)
(ii) RMCD (62.5 g/litre) and MeJA (100 micromoles/l)
(iii) MeJA (100 micromoles/l)
(iv) controls without RMCD or MeJA.

The experiments were carried out in triplicate in 250-ml flasks containing 100 ml of culture. After 96 hours, the cells and medium of each flask were collected separately, the cells were weighed and the total amount of tR in the extracellular medium was determined.

The result of the experiment is shown in Table F.

When the culture was stimulated with RMCD alone, an extracellular tR accumulation per unit of biomass was observed, although this was lower than that accumulated by other varieties - *Gamay, Monastrell Albina, Muscat Hamburg-* under similar conditions.

By contrast, the combination of RMCD with MeJA, resulted in an increase in the extracellular tR accumulation; since production with RMCD alone was very low, the increase was 37 times.

Therefore, the synergistic effect of RMCD and MeJA observed in the Monastrell Albina variety was also found in Monastrell Verde.

**TABLE F: tR production per unit of weight of Vitis vinifera Monastrell Verde cells after incubation for 96 hours.**

| **Treatment** | **tR (mg/g fresh weight)** | **Ratio area trans/cis** |
|---|---|---|
| Control | Not detected | -- |
| MJ | Not detected | -- |
| RMCD | 0.21± 0.08 | 1.83± 0.35 |
| RMCD + MJ | 7.84± 1.95 | 16.3± 0.94 |

| | | |
|---|---|---|
| RMCD at a concentration of 62.5 g/l; MJ: methyl jasmonate 100 micromolar. | | |

Bru and Pedreño (ES2190771) indicate that in *Monastrell verde* variety cultures, such as the one shown in this example, the cis-resveratrol isomer accumulates reaching levels close to those of trans-resveratrol when treated with RMCD. As shown in Figure 2A and 2B and Table F, this result was also obtained in the present example. However, as shown in Figure 2C and 2D and Table F, the inclusion of MeJA in the stimulation medium caused an additional effect to the increased production of resveratrol, being the dramatic increase in the trans/cis ratio, going from almost 2 times to 16 times. Since in other varieties the trans/cis ratio is very high, the effect had not been appreciated until using this variety.

### EXAMPLE 6: Elicitation of grapevine cells (Vitis vinifera var. Gamay) with RMCD and MeJA applied sequentially and in different order.

In the preceding examples, the elicitors are applied together at the start of incubation of the culture for stimulation. To determine whether the sequential addition of elicitors and order have any influence on the synergistic effect on tR production, two experiments were carried out.

In the first, elicitation was started with CD only and after a time methyl jasmonate was added. A culture treated with CD and MeJA from the beginning, and another culture treated with CD only were used as controls. As shown in Figure 3, the culture in which first CD was added and then MeJA, showed the synergistic effect after the addition of MeJA, reaching after 168 hours production levels equal to those of the control treated with CD and MeJA from the start.

In the second experiment, the culture with a density of 50% PCV was treated with 100 micromolar MeJA and after a while RMCD was added by 1:1 dilution of culture in a medium containing 125 g/l of RMCD and 100 micromolar MeJA.

Thus, the final concentrations of cells, RMCD and MeJA were 25% PCV, 62.5 g/l RMCD and 100 micromolar MeJA.

The controls used were:
a) a culture with a density of 50% PCV to which, at the time indicated, RMCD and MeJA were added by 1:1 dilution of culture in a medium containing 125 g/l of RMCD and 200 micromolar MeJA
b) a culture with a density of 50% PCV to which, at the time indicated, RMCD was added by 1:1 dilution of culture in a medium containing 125 g/l of RMCD.

No resveratrol production was detected during the 48-hour exposure to MeJA, nor in the controls used. As shown in Figure 4, from the time when the CDs were added (time 0 on the graph) resveratrol began to accumulate. The synergistic effect was clearly observed throughout the incubation time when comparing the accumulation in the controls (black circles) that only had CD with cultures containing CD and MeJA. In the latter, during the first 72 hours of incubation, no significant differences were noted between the levels accumulated in cultures pre-exposed to MeJA (triangles) and those unexposed (gray circles). From that time, pre-exposed cultures underwent deceleration in the accumulation of resveratrol, while those unexposed accelerated, almost doubling the levels of the pre-exposed cultures and nearly quadrupling those of controls without MeJA.

In conclusion, it was established that the synergistic effect observed was independent of the order in which the CD and MeJA elicitors were added.

### EXAMPLE 7: Elicitation of grapevine cells (Vitis vinifera var. Gamay) with RMCD, HPCD and MeJA.

To determine the effect of MeJA with a mixture of the two cyclodextrins, an experiment was carried out on cultures in the presence of:
a) culture with 31.5 g/l RMCD, 34.5 g/l HPCD and 100 micromolar MeJA
b) culture with 31.25 g/l RMCD, 34.5 g/l HPCD (control)
c) culture without elicitors (control)
d) culture with MeJA (control)

As shown in Table G the synergistic effect observed when dealing with individual CDs and MeJA was also observed when dealing with a mixture of the two CDs and MeJA. The data shown are the average of four biological replicates.

**TABLE G: T-resveratrol production per unit of weight of Vitis vinifera Gamay cells after 96 hours of incubation stimulated with MeJA and an approximately equimolar mixture of RMCD and HPCD.**

| **Treatment** | **tR (mg/g fresh weight)** |
|---|---|
| Control | Not detected |
| MJ | Not detected |
| RMCD + HPCD | 2.61± 0.33 |
| RMCD + HPCD + MJ | 6.56± 0.43 |

| | |
|---|---|
| RMCD at a concentration of 31.25 g/l. HPCD at a concentration of 34.5 g/l; MJ: methyl jasmonate 100 micromolar. | |

## Claims

1. Method to obtain resveratrol, which comprises:
a. adding cyclodextrins selected from the group comprising randomly methylated cyclodextrin or hydroxypropylated cyclodextrin and methyl jasmonate to a culture medium,
b. adding resveratrol-producing cells selected from the Vitis vinifera species to the culture medium obtained in step a),
c. incubating the cell culture medium of step b) and
d. separating the resveratrol obtained in step c) from the culture medium.

2. Method according to claim 1, further comprising:
e. purifying the resveratrol separated in step d)

3. Method according to any of claims 1 to 2, wherein the methyl jasmonate concentration is between 5 and 450 micromoles/litre of culture medium.

4. Method according to any of claims 1 to 2, wherein the methyl jasmonate concentration is between 50 and 150 micromoles/L culture 20 medium.

5. Method according to any of claims 1 to 4, wherein the randomly methylated cyclodextrin has a degree of methyl substitution of between 1 and 3.

6. Method according to claim 5, wherein the randomly methylated cyclodextrin has a degree of methyl substitution of between 1.5 and 2.

7. Method according to claim 6 wherein the randomly methylated cyclodextrin has a degree of methyl substitution of between 1.6 and 1.9.

8. Method according to any of claims 1 to 4, wherein the hydroxypropylated cyclodextrin has a degree of hydroxypropyl substitution of between 0.2 and 1.3.

9. Method according to claim 8, wherein the hydroxypropylated cyclodextrin has a degree of hydroxypropyl substitution of between 0.5 and 1.

10. Method according to claim 9, wherein the hydroxypropylated cyclodextrin has a degree of hydroxypropyl substitution of between 0.6 and 0.9.

11. Method according to any of claims 1 to 10, wherein the concentration of cyclodextrin is between 6.5 and 130 g/L culture medium.

12. Method according to claim 11, wherein the concentration of cyclodextrins is between 10 and 100 g/L culture medium.

13. Method according to claim 12 wherein the concentration of cyclodextrins is between 50 and 75 g/L culture medium.

14. Method according to any of claims 1 to 13, wherein the incubation time is between 4 and 288 hours.

## Patentansprüche

1. Verfahren zur Herstellung von Resveratrol, das Folgendes umfasst:
a. Versetzen eines Kulturmediums mit Cyclodextrinen, die ausgewählt werden aus der Gruppe bestehend aus willkürlich methyliertem Cyclodextrin oder hydroxypropyliertem Cyclodextrin und Methyljasmonat,
b. Versetzen des in Schritt a) erhaltenen Kulturmediums mit Resveratrol produzierenden Zellen, die aus den Arten Vitis vinifera ausgewählt werden,
c. Inkubieren des Zellkulturmediums aus Schritt b) und
d. Trennen des in Schritt c) erhaltenen Resveratrols vom Kulturmedium.

2. Verfahren nach Anspruch 1, das weiterhin umfasst:
e. Reinigen des in Schritt d) abgetrennten Resveratrols.

3. Verfahren nach einem der Ansprüche 1 bis 2, wobei die Konzentration von Methyljasmonat zwischen 5 und 450 Mikromol/Liter Kulturmedium liegt.

4. Verfahren nach einem der Ansprüche 1 bis 2, wobei die Konzentration von Methyljasmonat zwischen 50 und 150 Mikromol/l Kulturmedium liegt.

5. Verfahren nach einem der Ansprüche 1 bis 4, wobei das willkürlich methylierte Cyclodextrin einen Methyl-Substitutionsgrad von 1 bis 3 aufweist.

6. Verfahren nach Anspruch 5, wobei das willkürlich methylierte Cyclodextrin einen Methyl-Substitutionsgrad von 1,5 bis 2 aufweist.

7. Verfahren nach Anspruch 6, wobei das willkürlich methylierte Cyclodextrin einen Methyl-Substitutionsgrad von 1,6 bis 1,9 aufweist.

8. Verfahren nach einem der Ansprüche 1 bis 4, wobei das hydroxypropylierte Cyclodextrin einen Hydroxypropyl-Substitutionsgrad von 0,2 bis 1,3 aufweist.

9. Verfahren nach Anspruch 8, wobei das hydroxypropylierte Cyclodextrin einen Hydroxypropyl-Substitutionsgrad von 0,5 bis 1 aufweist.

10. Verfahren nach Anspruch 9, wobei das hydroxypropylierte Cyclodextrin einen Hydroxypropyl-Substitutionsgrad von 0,6 bis 0,9 aufweist.

11. Verfahren nach einem der Ansprüche 1 bis 10, wobei die Konzentration von Cyclodextrin zwischen 6,5 und 130 g/l Kulturmedium liegt.

12. Verfahren nach Anspruch 11, wobei die Konzentration von Cyclodextrin zwischen 10 und 100 g/l Kulturmedium liegt.

13. Verfahren nach Anspruch 12, wobei die Konzentration von Cyclodextrin zwischen 50 und 75 g/l Kulturmedium liegt.

14. Verfahren nach einem der Ansprüche 1 bis 13, wobei die Inkubationszeit zwischen 4 und 288 Stunden beträgt.

## Revendications

1. Méthode de production de resvératrol, comprenant :
a. l'ajout de cyclodextrines sélectionnées dans le groupe comprenant une cyclodextrine méthylée aléatoirement ou une cyclodextrine hydroxypropylée et du méthyl jasmonate dans un milieu de culture,
b. l'ajout de cellules productrices de resvératrol sélectionnées à partir de l'espèce Vitis vinifera dans le milieu de culture obtenu à l'étape a),
c. l'incubation du milieu de culture cellulaire de l'étape b) et
d. la séparation du resvératrol obtenu à l'étape c) dans le milieu de culture.

2. Méthode conforme à la revendication 1, comprenant de plus :
e. la purification du resvératrol séparé à l'étape d)

3. Méthode conforme à l'une des revendications 1 à 2, dans laquelle la concentration en méthyl jasmonate est de 5 à 450 micromoles/litre de milieu de culture.

4. Méthode conforme à l'une des revendications 1 à 2, dans laquelle la concentration en méthyl jasmonate est de 50 à 150 micromoles/litre de milieu de culture 20.

5. Méthode conforme à l'une des revendications 1 à 4, dans laquelle la cyclodextrine méthylée aléatoirement présente un degré de substitution du méthyle de 1 à 3.

6. Méthode conforme à la revendication 5, dans laquelle la cyclodextrine méthylée aléatoirement présente un degré de substitution du méthyle de 1,5 à 2.

7. Méthode conforme à la revendication 6, dans laquelle la cyclodextrine méthylée aléatoirement présente un degré de substitution du méthyle de 1,6 à 1,9.

8. Méthode conforme à l'une des revendications 1 à 4, dans laquelle la cyclodextrine hydroxypropylée présente un degré de substitution de l'hydroxypropyle de 0,2 à 1,3.

9. Méthode conforme à la revendication 8, dans laquelle la cyclodextrine hydroxypropylée présente un degré de substitution de l'hydroxypropyle de 0,5 à 1.

10. Méthode conforme à la revendication 9, dans laquelle la cyclodextrine hydroxypropylée présente un degré de substitution de l'hydroxypropyle de 0,6 à 0,9.

11. Méthode conforme à l'une des revendications 1 à 10, dans laquelle la concentration en cyclodextrines est de 6,5 à 130 g/L de milieu de culture.

12. Méthode conforme à la revendication 11, dans laquelle la concentration en cyclodextrines est de 10 à 100 g/L de milieu de culture.

13. Méthode conforme à la revendication 12, dans laquelle la concentration en cyclodextrines est de 50 à 75 g/L de milieu de culture.

14. Méthode conforme à l'une des revendications 1 à 13, dans laquelle la durée d'incubation est de 4 à 288 heures.
